# FASCICULE DE BREVET EUROPEEN

(11) **EP 3 796 852 B1**
(45) Date de publication et mention de la délivrance du brevet: **27.08.2025**
(21) Numéro de dépôt: 19740613.5
(22) Date de dépôt: 22.05.2019
(51) Int. Cl.: A61B 17/34, A61B 17/3205, A61B 17/00, A61M 60/178, A61M 60/216, A61M 60/863

(54) **DISPOSITIF DE CAROTTAGE ET ENSEMBLE DE CAROTTAGE ET D'INTRODUCTION COMPRENANT UN TEL DISPOSITIF**
CORING-VORRICHTUNG UND CORING- UND EINFÜHRUNGSANORDNUNG MIT SOLCH EINER VORRICHTUNG
CORING DEVICE AND CORING AND INSERTION ASSEMBLY COMPRISING SUCH A DEVICE

(30) Priorité: 22.05.2018 FR 1854238
(43) Date de publication de la demande: 31.03.2021
(73) Titulaire: Fineheart, 33600 Pessac (FR)
(72) Inventeur: MASCARELL, Arnaud, 37250 MONTBAZON (FR); GARRIGUE, Stéphane, 33130 BEGLES (FR)
(74) Mandataire: Plasseraud IP
(86) Numéro de dépôt international: PCT/FR2019/051161
(87) Numéro de publication internationale: WO 2019/224477

(56) Documents cités:
- WO-A1-2014/117087
- US-A1- 2007 167 968
- US-A1- 2008 097 595
- US-A1- 2009 082 778
- US-A1- 2012 296 358
- US-A1- 2014 058 431
- US-A1- 2016 121 033

## Description

### ARRIERE-PLAN DE L'INVENTION

### Domaine de l'invention

La présente invention concerne un dispositif de carottage pour réaliser une ouverture dans une paroi tissulaire, notamment en vue de la pose d'une pompe cardiaque sur un cœur battant.

Elle concerne également un ensemble pour le carottage d'une paroi d'un cœur battant et l'introduction d'une portion d'un dispositif d'ancrage d'une pompe cardiaque au travers de l'ouverture réalisée par carottage.

### Arrière-plan technologique

L'insuffisance cardiaque (IC), est un état pathologique dans lequel le cœur d'un patient présente une incapacité à fournir un débit sanguin nécessaire aux besoins métaboliques de l'organisme.

Il est connu pour traiter l'insuffisance cardiaque d'implanter un dispositif d'assistance ventriculaire (DAV), qui est une pompe cardiaque artificielle.

Cette pompe mécanique ne remplace pas le cœur qui continue à fonctionner, mais apporte une aide au ventricule affaibli afin d'accroître le débit sanguin de façon adaptée aux besoins de l'individu.

Cette assistance peut être temporaire dans l'attente d'un greffon disponible pour réaliser une transplantation cardiaque.

Cependant, on observe une proportion significative de patients qui ne recevront pas un tel greffon, soit parce qu'ils ne peuvent être candidats à une telle transplantation, par exemple en raison d'une insuffisance cardiaque sévère, soit parce qu'aucun greffon adapté n'est disponible pour ces patients.

Dans ce cas, l'assistance ventriculaire est utilisée en destination, c'est-à-dire que la pompe cardiaque artificielle est implantée à long terme.

Ces pompes cardiaques font donc l'objet d'intenses recherches visant à améliorer la survie et la qualité de vie des patients présentant une insuffisance cardiaque.

De nombreuses avancées ont été réalisées ces dernières années et on connaît aujourd'hui des dispositifs d'assistance ventriculaire plus compacts, silencieux et présentant une durée de service accrue.

Les pompes cardiaques implantables de l'état de l'art sont ainsi typiquement équipées d'un moteur électrique intégré pour assurer leur fonctionnement, la vitesse de rotation de la pompe fournissant la force nécessaire pour faire circuler le sang depuis le ventricule affaibli vers la circulation corporelle.

On connaît des systèmes d'implantation de telles pompes dans une ouverture d'une paroi ventriculaire.

Ces systèmes d'implantation comprennent généralement une portion tubulaire aux extrémités de laquelle sont placés, ou formés, des collerettes destinées à être plaquées chacune contre une face opposée de la paroi ventriculaire après introduction de la portion tubulaire dans une ouverture réalisée dans cette paroi ventriculaire avec un dispositif de carottage.

Ces collerettes permettent ainsi de maintenir en position cette portion tubulaire creuse, laquelle définit alors un conduit ouvert traversant la paroi ventriculaire. Le document US 2007/167968 A1 montre un tel système.

A l'extrémité, placée à l'extérieur du cœur, de cette portion tubulaire, est introduite une pompe aspirative qui une fois installée, assure le renvoi du sang présent dans le ventricule vers la circulation corporelle.

Bien que représentant un progrès certain pour la qualité de vie d'un patient souffrant d'insuffisance cardiaque, de nombreux inconvénients sont encore constatés.

Notamment, la réalisation de cette ouverture sur un cœur battant pour minimiser les effets secondaires de l'intervention sur le patient peut entraîner des pertes de sang importantes durant l'implantation de la pompe cardiaque.

Une grande dextérité et une rapidité d'action des praticiens sont en conséquence, des facteurs importants de réussite, ce qui est source de stress pour l'équipe réalisant l'intervention.

De plus, les dimensions des collerettes étant réduites pour faciliter leur passage au travers de l'ouverture réalisée dans la paroi ventriculaire, la tenue mécanique du système d'implantation est limitée. Il n'autorise pas, par exemple, l'application d'efforts importants sur celui-ci une fois en place sur la paroi ventriculaire.

En outre, il est souhaitable que l'extrémité d'éjection du sang de la pompe soit centrée sur la valve cardiaque pour assurer un flux de sang éjecté optimal.

Le positionnement de l'ouverture dans la paroi ventriculaire participe alors des performances réelles de la pompe cardiaque dans l'éjection du sang dans l'aorte.

Or, on constate qu'en l'absence de repères, cette ouverture n'est pas toujours bien positionnée au regard de la valve cardiaque. Le flux de sang éjecté n'est ainsi pas idéal.

Il existe donc un besoin pressant pour un dispositif de carottage dont la conception originale surmonte les inconvénients décrits ci-dessus.

### Objet de l'invention

L'ensemble de la présente invention est défini dans la revendication 1. Des modes de réalisation sont définis dans les revendications dépendantes.

La présente invention vise à pallier les inconvénients de l'art antérieur et à répondre aux contraintes ci-dessus énoncées en proposant un ensemble de carottage d'une paroi tissulaire, simple dans sa conception et dans son mode opératoire, fiable et d'un maniement particulièrement aisé pour un praticien.

Un autre objet de la présente invention est un tel ensemble de carottage offrant plusieurs fonctions et, notamment, étant configuré pour recevoir, supporter et un assurer un centrage d'un outil d'assistance à la pose d'une partie de dispositif d'ancrage par rapport à l'ouverture réalisée par carottage.

Encore un objet de la présente invention est un tel ensemble de carottage permettant d'orienter la tête de carottage lors de la réalisation de l'ouverture dans la paroi tissulaire pour assurer un positionnement optimal de l'ouverture réalisée dans la paroi tissulaire au regard de la valve cardiaque.

La présente invention vise également un ensemble pour le carottage d'une paroi d'un cœur battant et l'introduction d'une portion d'un dispositif d'ancrage d'une pompe cardiaque au travers de l'ouverture réalisée par carottage, assurant une pose facilitée de cette portion de dispositif d'ancrage et autorisant, en conséquence, une maximisation de ses dimensions.

Un autre objet de la présente invention est un tel ensemble limitant les risques de perte de sang pour le patient.

### BREVE DESCRIPTION DE L'INVENTION

A cet effet, l'invention concerne un dispositif de carottage pour réaliser une ouverture dans une paroi tissulaire, comprenant :
- un corps ayant un axe longitudinal, ledit corps ayant une extrémité proximale et une extrémité distale, ledit corps comportant un logement interne,
- une tête de carottage comportant une lame de transsection, cette tête étant mobile entre une position de repos et une position actionnée, ladite tête de carottage ayant un axe de carottage,
- un mécanisme d'actionnement pour déplacer ladite tête entre lesdites positions, ledit mécanisme comportant une tige d'actionnement à une extrémité de laquelle est placée ladite tête mobile, et
- l'extrémité distale dudit corps forme un support d'outil chirurgical, ou accessoire chirurgical, ce support d'outil étant configuré pour
   * d'une part, recevoir par emboîtement un outil chirurgical creux et supporter cet outil, cet outil chirurgical entourant alors l'extrémité distale dudit corps lorsqu'il est placé sur ledit support d'outil, en étant coaxial avec ledit axe de carottage, et
   * d'autre part, permettre un guidage linéaire, ou sensiblement linéaire, dudit outil chirurgical lors de son déplacement vers l'extérieur du dispositif de carottage.

De manière avantageuse, un tel dispositif de carottage permet non seulement de réaliser une ouverture dans une paroi tissulaire mais également d'introduire dans l'ouverture réalisée, l'extrémité distale d'un outil chirurgical, par exemple un outil d'assistance à la pose d'une partie de dispositif d'ancrage, ce qui confère un gain de temps, un confort accru pour l'équipe chirurgicale et une sécurité renforcée pour le patient.

Il n'est en effet pas nécessaire qu'un praticien, autre que celui ayant réalisé l'ouverture, intervienne rapidement après réalisation de celle-ci pour y introduire l'outil ou accessoire chirurgical et il n'y a aucun changement de matériel opératoire.

Un tel dispositif permet également d'introduire cet outil chirurgical dans l'ouverture réalisée, car la configuration de ce support d'outil assure un alignement, ou une correspondance, de cette ouverture et de l'extrémité distale de l'outil chirurgical, laquelle est configurée pour passer au travers de cette ouverture.

Un tel dispositif de carottage est particulièrement utile pour réaliser une ouverture dans une paroi ventriculaire d'un cœur battant en vue de la pose et de l'assemblage d'une bague d'ancrage, encore connue sous le nom de dispositif d'ancrage ou de fixation, d'une pompe cardiaque dans cette ouverture.

Une telle bague d'ancrage comprend typiquement une partie de bague comportant un corps creux à l'extrémité duquel est placé une membrane auto-expansible entre une première configuration, dite déformée, dans laquelle elle présente une forme tubulaire, ou essentiellement tubulaire, et une seconde configuration, dite initiale, dans laquelle elle définit une bride, ou collerette, s'étendant radialement, ou sensiblement radialement, à partir dudit corps creux, cette bride étant destinée à venir en aboutement contre une face intérieure de ladite paroi ventriculaire du cœur battant.

L'outil chirurgical est alors avantageusement un corps creux de forme générale cylindrique configuré pour être reçu dans l'ouverture réalisée dans la paroi ventriculaire et maintenir ouverte cette ouverture dans ses dimensions maximales pour faciliter l'introduction d'au moins la membrane auto-expansible dans la chambre ventriculaire.

Dans différents modes de réalisation particuliers de ce dispositif, chacun ayant ses avantages particuliers et susceptibles de nombreuses combinaisons techniques possibles:
- ce dispositif de carottage comporte une lumière s'étendant à travers au moins l'extrémité proximale dudit corps, ladite tige d'actionnement et ladite tête de carottage pour le passage d'un élément de guidage souple.

La mise en œuvre d'un élément de guidage souple tel qu'un câble de guidage, passant au travers de la paroi ventriculaire et de la valve cardiaque correspondante, par exemple la valve aortique, permet d'orienter l'ouverture réalisée dans la paroi ventriculaire sur cette valve cardiaque.

De manière avantageuse, une telle orientation assure ab initio un centrage optimal du corps de pompe sur cette valve cardiaque, et donc de bonne performance en matière d'éjection du sang au travers de la valve cardiaque.

De préférence, cette lumière supporte un tel élément de guidage souple.
- ledit support d'outil comporte une paroi périphérique externe qui est tubulaire et de diamètre égal ou sensiblement égal au diamètre de ladite lame de transsection.

On entend ici par « sensiblement égal » que ce diamètre peut varier de quelques pourcents par rapport au diamètre de l'ouverture réalisée dans la paroi ventriculaire.

L'outil chirurgical creux est alors au moins en partie de forme tubulaire ou sensiblement tubulaire, en présentant un diamètre intérieur égal ou sensiblement égal au diamètre de ladite lame de transsection de sorte que cet outil chirurgical peut coulisser le long de ladite tête mobile lorsqu'il est introduit sur le, ou sortie du, support d'outil.
- ladite paroi tissulaire étant une paroi d'un cœur battant destiné à recevoir un dispositif d'ancrage d'une pompe cardiaque comprenant une bride, ou collerette, destinée à être plaquée contre la surface extérieure de ladite paroi du cœur battant, la longueur dudit support d'outil est au moins égale à la somme de l'épaisseur de ladite paroi tissulaire et de l'épaisseur, ou dimension longitudinale, de ladite bride, ou collerette, destinée à être placée contre la surface extérieure de la paroi dudit cœur.
- le dispositif comporte une partie d'un moyen détrompeur, ledit outil chirurgical étant destiné à porter la partie complémentaire de ce moyen détrompeur, pour orienter ledit outil chirurgical sur ledit support d'outil.

A titre purement illustratif, le dispositif peut comporter un ergot destiné à être reçu dans un logement correspondant placé sur la tranche de l'extrémité proximale de l'outil chirurgical.
- le dispositif comporte une butée pour stopper le déplacement dudit outil chirurgical le long de l'axe longitudinal dudit corps et en direction de ladite extrémité proximale afin que l'outil chirurgical reste positionné sur ledit support d'outil.
- ledit corps comporte deux parties de corps emboîtées l'une dans l'autre, une première partie de corps étant mobile en translation par rapport à l'autre partie de corps de sorte que son déplacement dans et hors de l'autre partie de corps provoque le déplacement en translation de ladite tête de carottage.

De préférence, chaque partie de corps comporte une poignée de préhension. De manière avantageuse, ledit mécanisme d'actionnement comprend un chariot de guidage portant ladite tige d'actionnement et reçu dans ledit logement interne, ce logement étant configuré pour former un rail de guidage en translation dudit chariot de guidage, ledit chariot de guidage étant solidaire de ladite première partie de corps mobile en translation.

La présente invention concerne également un ensemble de pose d'un outil ou d'un accessoire chirurgical dans une paroi tissulaire. Selon l'invention, cet ensemble comprend :
- un dispositif de carottage tel que décrit précédemment, et
- un outil ou accessoire chirurgical creux configuré pour être emboîté sur l'extrémité distale dudit dispositif de carottage tout en laissant ladite tête de carottage dégagée.

Selon l'invention, ledit outil ou accessoire chirurgical comporte à son extrémité distale, une portion tubulaire ou sensiblement tubulaire configurée pour maintenir la forme et le diamètre de l'ouverture réalisée par carottage dans la paroi tissulaire.

De préférence, cette portion tubulaire ou sensiblement tubulaire a une longueur au moins égale à l'épaisseur de la paroi tissulaire.

Selon un mode de réalisation de l'ensemble de l'invention, le corps du dispositif de carottage comportant une partie d'un moyen détrompeur, cet outil ou accessoire chirurgical comporte une partie complémentaire de ce moyen détrompeur, pour orienter ledit outil ou accessoire chirurgical sur ledit support d'outil.

Selon encore un autre mode de réalisation, ledit outil ou accessoire chirurgical étant un outil d'assistance à la pose d'un dispositif d'ancrage d'une pompe cardiaque au travers d'une ouverture réalisée par carottage dans la paroi d'un cœur, ledit outil d'assistance creux délimite un canal interne pour le passage dudit dispositif d'ancrage, dont le diamètre est sensiblement égal au diamètre de cette ouverture.

De manière avantageuse, ladite portion tubulaire ou sensiblement tubulaire de cet outil d'assistance a une longueur au moins égale à la somme de l'épaisseur de la paroi dudit cœur et de l'épaisseur, ou dimension longitudinale, d'une bride, ou collerette, destinée à être assemblée à la surface extérieure de la paroi dudit cœur.

De manière préférentielle, le corps du dispositif de carottage comportant une partie d'un moyen détrompeur, cet outil d'assistance comporte une partie complémentaire de ce moyen détrompeur, pour orienter ledit outil chirurgical sur ledit support d'outil selon une configuration prédéfinie. Par exemple, cet agencement prédéfini de l'outil chirurgical assure son orientation par rapport à une bride, ou collerette, assemblée contre la surface extérieure de la paroi dudit cœur. De préférence, cette bride, ou collerette, destinée à être placée contre la surface extérieure de la paroi dudit cœur comprenant au moins une saillie, ou téton, placée sur sa face latérale, ledit outil chirurgical comporte pour chaque saillie, une rainure débouchant destinée à recevoir ladite saillie, ou téton, correspondante, ladite rainure étant configurée pour assurer par rotation dudit outil chirurgical autour de l'axe de carottage, un verrouillage dudit outil chirurgical sur ladite bride, ou collerette

Selon encore un autre mode de réalisation de l'ensemble de l'invention, cet ensemble comporte un dispositif de poussée allongé comportant à son extrémité distale une tête support d'une partie de dispositif d'ancrage comportant ladite bride, ou collerette, dans sa configuration déformée, ladite tête présentant un moyen d'assemblage tel qu'un filetage, avec ladite partie de dispositif d'ancrage et cette dernière comprenant un moyen d'assemblage complémentaire pour assurer l'assemblage de ces éléments, ledit dispositif de poussée allongé étant configuré pour traverser ledit outil chirurgical de sorte que ladite bride, ou collerette de la bague d'ancrage puisse traverser l'ouverture réalisée dans la paroi dudit cœur.

De préférence, ce dispositif de poussée a la forme d'un tube ou d'une tige comportant une lumière pour recevoir l'élément de guidage souple.

### BREVE DESCRIPTION DES DESSINS

D'autres avantages, buts et caractéristiques particulières de la présente invention ressortiront de la description qui va suivre, faite, dans un but explicatif et nullement limitatif, en regard des dessins annexés, dans lesquels:
- la Figure 1 représente schématiquement un dispositif de carottage pour réaliser une ouverture dans une paroi ventriculaire d'un cœur battant selon un mode de réalisation particulier de la présente invention ;
- la Figure 2 montre le dispositif de carottage de la Fig. 1, un accessoire d'assistance à la pose d'une bague d'ancrage étant reçu à l'extrémité distale de ce dernier pour définir un ensemble de carottage et d'introduction d'une partie d'une bague d'ancrage d'un dispositif d'assistance ventriculaire ;
- la Figure 3 est une vue partielle et élargie de l'ensemble illustré à la Fig. 2 montrant un moyen détrompeur pour orienter l'accessoire d'assistance à la pose ;
- la Figure 4 est une représentation schématique de l'ensemble de la Fig. 2, la tête de carottage étant dans sa position actionnée pour son introduction dans un petit orifice, préalablement réalisé avec un outil coupant dans la paroi ventriculaire du cœur ;
- la Figure 5 est une vue en perspective et en coupe de l'ensemble de la Fig. 2 juste après réalisation de la découpe dans la paroi ventriculaire ;
- la Figure 6 est une vue partielle et de dessus de l'ensemble de la Fig. 2, lors de l'assemblage de l'accessoire d'assistance avec la bride préalablement assemblée à la surface extérieure de ladite paroi ventriculaire, l'extrémité distale de l'accessoire d'assistance étant alors insérée dans l'ouverture réalisée ;
- la Figure 7 est une vue en coupe longitudinale de l'ensemble de la Fig. 2 et de l'apex du cœur, le dispositif de carottage étant en cours de retrait, l'extrémité distale de l'accessoire d'assistance, insérée dans l'ouverture réalisée, étant apparent ;
- la Figure 8 est une vue en perspective de l'accessoire d'assistance de la Fig. 2 assemblé à une bride solidaire de l'apex du cœur, une partie d'une bague d'ancrage comportant une bride dans sa configuration déformée étant introduite dans cet accessoire d'assistance au moyen d'un dispositif de poussée ;

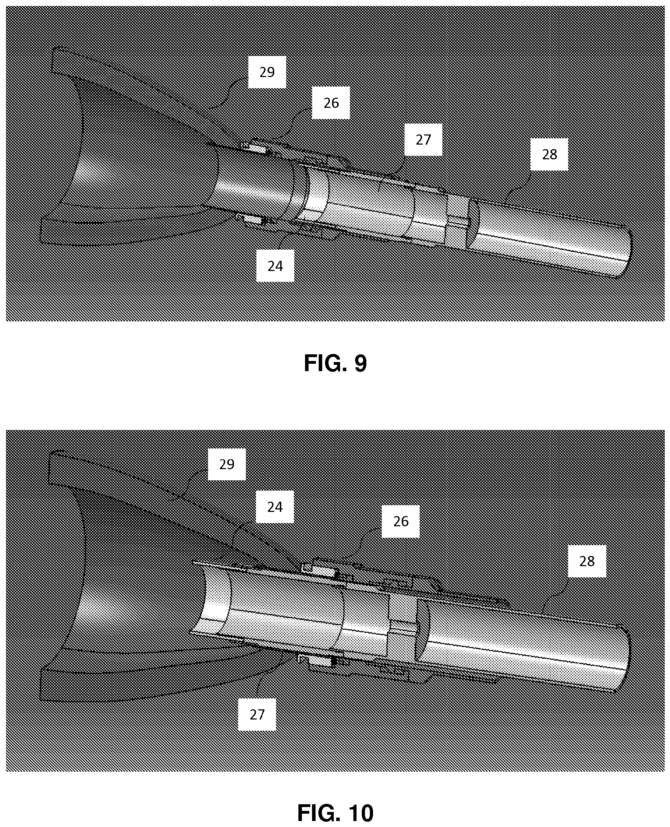

- la Figure 9 est une vue en coupe longitudinale des éléments illustrés à la Fig. 8 ;
- la Figure 10 est une vue en coupe longitudinale des éléments illustrés à la Fig. 8, la bride dans sa configuration déformée étant introduite dans la chambre ventriculaire au moyen du dispositif de poussée ;

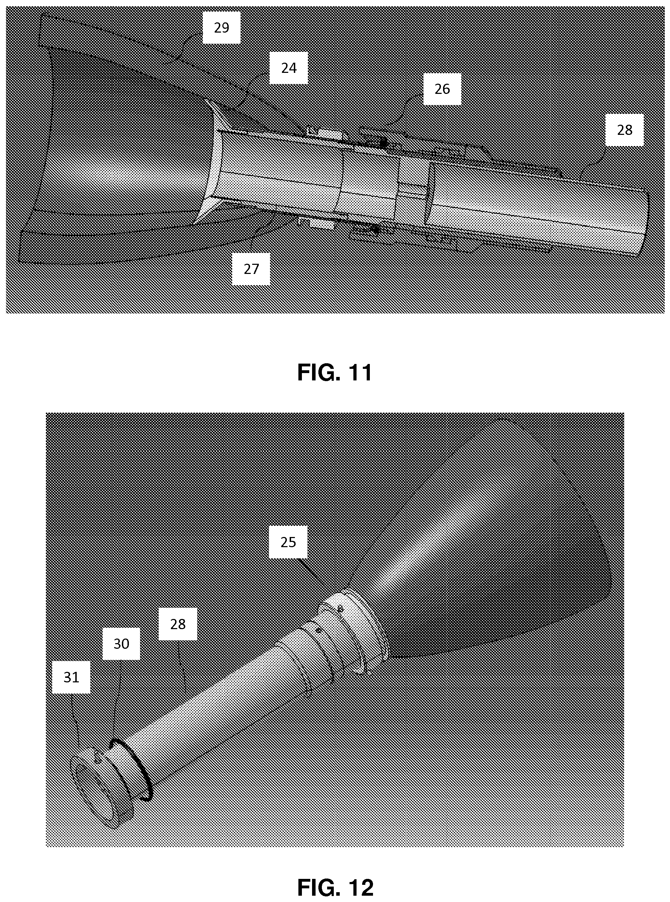

- la Figure 11 est une vue en coupe longitudinale des éléments illustrés à la Fig. 8, la bride étant dans sa configuration initiale pour définir une collerette, l'accessoire d'assistance à la pose ayant été désassemblé de la bride montée à la surface extérieure de la paroi ventriculaire et étant en cours de retrait ;
- la Figure 12 est une vue en perspective des éléments illustrés à la Fig. 8, l'accessoire d'assistance ayant été retiré, un joint d'étanchéité et un écrou étant engagés sur le dispositif de poussée pour bloquer en position de la bague d'ancrage ;
- la Figure 13 est une vue en perspective de la bague d'ancrage de la Fig. 12 montée dans son ouverture et supportant une pompe cardiaque.

### DESCRIPTION DETAILLEE DE MODE DE REALISATION DE L'INVENTION

Tout d'abord, on note que les figures ne sont pas à l'échelle.

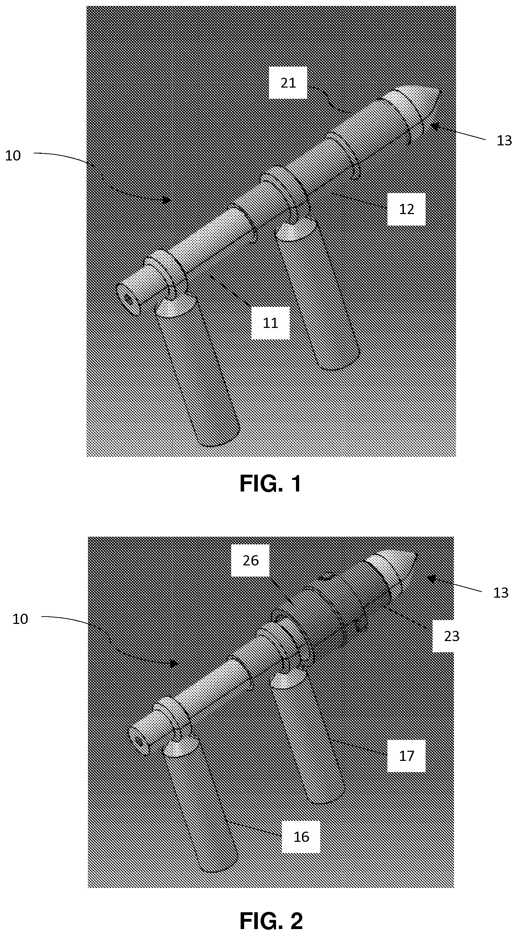

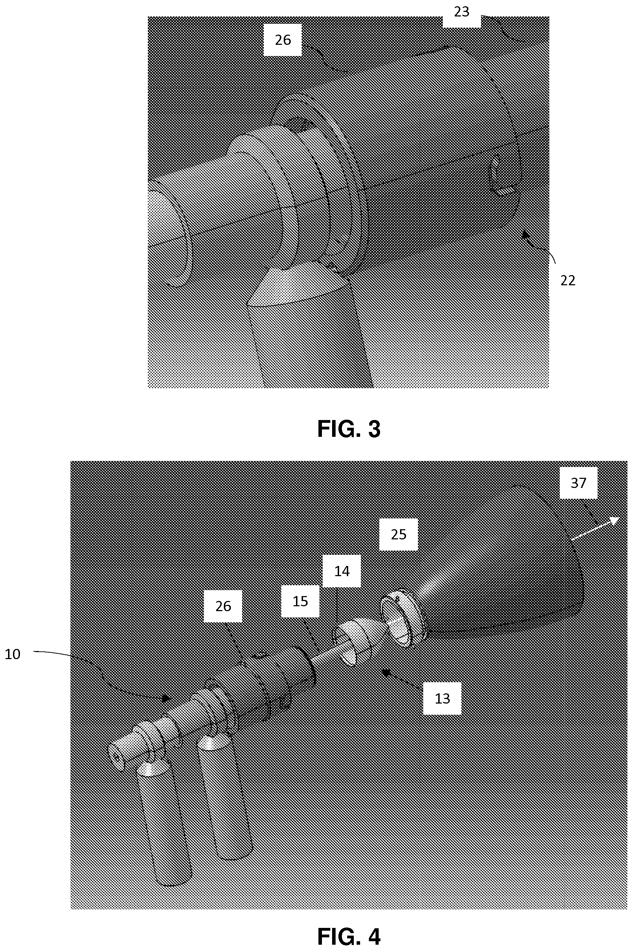

Les Figures 1 à 7 montrent de manière schématique un dispositif de carottage 10 pour réaliser une ouverture dans la paroi ventriculaire d'un cœur battant, selon un mode de réalisation particulier de la présente invention.

Ce dispositif de carottage 10 comporte deux parties de corps 11, 12 qui sont emboitées l'une dans l'autre de sorte qu'une de ces parties est montée en coulissement dans l'autre partie de corps. Ce dispositif présente une extrémité proximale et une extrémité distale.

Dans le contexte de la présente invention, le terme « proximal » signifie la position la plus proche du professionnel de santé, ou du praticien, tandis que le terme « distal » doit ici être entendu comme signifiant le plus éloigné de ce professionnel. En d'autres termes, l'extrémité distale d'une pièce est l'extrémité qui serait la première engagée dans le cœur battant tandis que son extrémité proximale serait la dernière extrémité à y être engagée.

Ce dispositif de carottage 10 comprend une tête de carottage 13 comportant une pointe ayant une forme frustro-conique, à son extrémité distale, pour faciliter son insertion au travers d'une incision réalisée avec un outil coupant tel qu'un scalpel, dans la paroi ventriculaire de ce cœur battant. Cette tête 13 comporte également une lame de transsection 14 placée à son extrémité proximale. Cette lame de transsection 14 présente une section transverse droite de forme circulaire mais pourrait présenter d'autres formes appropriées telles qu'ovale.

Cette tête de carottage 13 est mobile entre une position de repos, dans laquelle la lame de transsection 14 est plaquée contre, ou est placée à proximité de, l'extrémité distale du corps principal du dispositif de carottage 10, et une position actionnée dans laquelle la lame de transsection 14 est placée à distance de l'extrémité distale du corps principal du dispositif de carottage 10.

Plus précisément, cette tête de carottage 13 est actionnée par un mécanisme d'actionnement comprenant une tige d'actionnement 15, à l'extrémité de laquelle cette tête 13 est montée, cette tige 15 étant couplée à un logement interne du corps principal du dispositif de carottage 10.

Ce corps principal comporte ici deux parties 11, 12 de corps emboîtées l'une dans l'autre, une première partie de corps étant mobile en translation par rapport à l'autre partie de corps de sorte que son déplacement dans et hors de l'autre partie de corps provoque le déplacement en translation de ladite tête de carottage 13.

Chaque partie de corps comporte une poignée 16, 17 de préhension ayant un axe longitudinal Z. Ces poignées 16, 17 sont ici arrangées de sorte que leur axe longitudinal est perpendiculaire à l'axe longitudinal défini par le corps du dispositif de carottage 10.

Ainsi, le praticien peut tenir d'une première main, une poignée 16 d'une première partie 11 de corps tandis que son autre main tient la poignée 17 de l'autre partie 12 de corps pour déplacer aisément ces deux parties de corps l'une par rapport à l'autre et ainsi provoquer le déplacement de la tête de carottage 13.

Avantageusement, l'axe géométrique ou axe de carottage du dispositif de carottage 10 est celui de la tige d'actionnement 15 sur laquelle est centrée la tête de carottage 13.

Le mécanisme d'actionnement comprend également un chariot de guidage 18 supportant la tige d'actionnement 15 et reçu dans le logement interne 19 du corps principal. La tige d'actionnement 15 est avantageusement centrée.

Le chariot de guidage 18 étant solidaire de la première partie de corps mobile en translation, ce logement interne 19 définit un rail de guidage en translation de ce chariot de guidage.

Une lumière 20 s'étend à travers l'extrémité proximale du corps principal du dispositif de carottage 10, la tige d'actionnement 15 et la tête de carottage 13 pour permettre le passage d'un guide 37. Ce guide 37 est destiné au guidage, notamment l'orientation, de la tête de carottage 13 lors de son approche de la paroi ventriculaire en vue de réaliser une ouverture par carottage.

Plus précisément, ce guide 37 est destiné à passer à travers la valve aortique pour guider le dispositif de carottage 13 et fixer ainsi l'orientation de l'ouverture à réaliser par carottage par rapport à la localisation anatomique de la valve aortique (ou de la valve pulmonaire si ce dispositif de carottage 13 est utilisé pour le ventricule droit).

De manière avantageuse, l'extrémité distale du corps principal du dispositif de carottage 10 forme également un support d'accessoire 21.

Ce support d'accessoire 21 comporte une paroi périphérique externe tubulaire dont le diamètre extérieur est égal au diamètre de la lame de transsection 14.

Un accessoire d'assistance 22 à la pose d'une partie d'une bague d'ancrage d'une pompe cardiaque, est reçu par emboitement sur ce support d'accessoire 21. Cet accessoire d'assistance 22 est creux et comporte un fourreau 23 s'étendant d'une portion intermédiaire de celui-ci vers son extrémité distale.

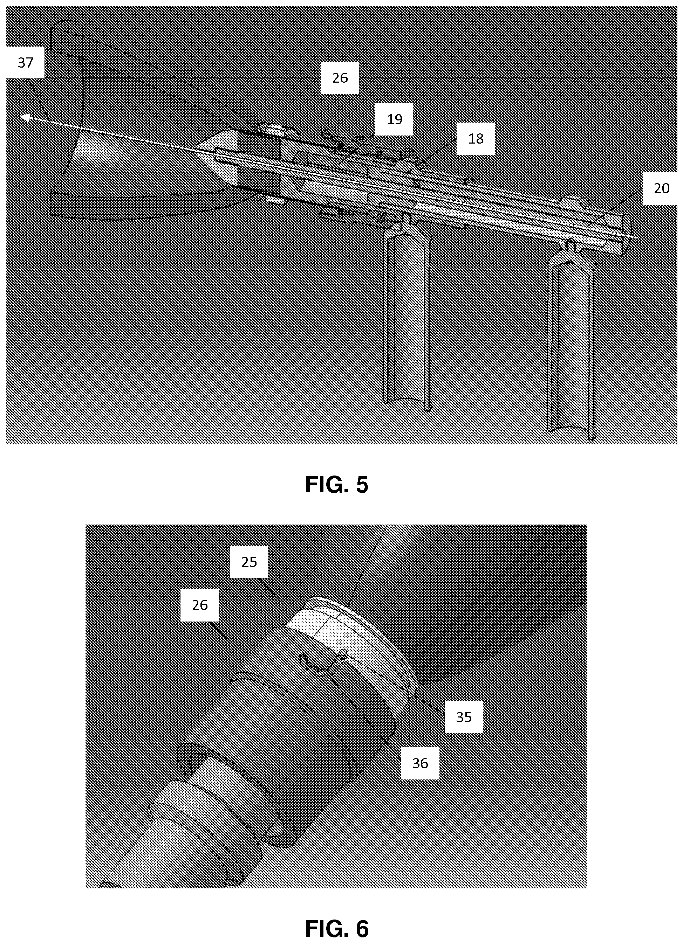

Comme représenté sur la Fig. 5, tandis que la tête de carottage 13 est encore partiellement placée dans la chambre ventriculaire, le praticien peut introduire dans l'ouverture réalisée dans la paroi ventriculaire, une partie de l'accessoire d'assistance 22, notamment son fourreau 23, en le déplaçant en translation sur le support d'accessoire 21, lequel assure un guidage linéaire.

Cet outil d'assistance étant réalisé dans un matériau rigide tel qu'en titane, son fourreau 23 maintient l'ouverture dans son extension maximale et délimite par son canal intérieur, un chemin pour le libre passage d'une partie de bague d'ancrage comprenant une portion tubulaire et à l'extrémité distale de cette dernière, une membrane auto-expansible 24 dans sa première configuration, dite déformée, dans laquelle celle-ci présente une forme tubulaire, ou essentiellement tubulaire. Cette membrane auto-expansible 24 est par exemple, réalisée en nitinol.

Une autre bride, ou seconde bride 25, de la bague d'ancrage ayant été préalablement assemblée, par exemple par suture, avec la surface extérieure de la paroi ventriculaire, l'accessoire d'assistance 22 comporte à son extrémité proximale, une tête de liaison 26 cylindrique creuse formant saillie du fourreau 23 en entourant en partie ce dernier. Cette tête de liaison 26 est destinée à être assemblée à cette seconde bride 25 pour réaliser un couplage amovible de l'accessoire avec l'ensemble seconde bride/apex du cœur. Cette seconde bride 25 est par exemple réalisée en dacron.

Comme illustré à la Figure 6, cette seconde bride 25 étant plaquée contre la surface extérieure de la paroi ventriculaire, elle comprend au moins un téton 35 positionné sur sa paroi latérale, la tête de liaison 26 de l'accessoire d'assistance 22 comportant pour chaque téton 35, une rainure 36 de couplage placée sur sa tranche et configurée pour recevoir ce téton correspondant. Plus précisément, chaque rainure 36 débouche à l'extrémité distale de cette tête de liaison 26 de sorte que cette dernière recouvrant partiellement la seconde bride 25 de la bague d'ancrage, chaque téton 35 est engagé dans sa rainure correspondante.

Chaque rainure 36 présente en outre une même forme incurvée de sorte que, par rotation de l'accessoire d'assistance 22 autour de l'axe de carottage, un verrouillage de cet accessoire d'assistance 22 sur la seconde bride 25 est obtenu.

Cet accessoire d'assistance 22 comporte également un élément d'étanchéité pour assurer l'étanchéité de l'assemblage tête de liaison 26/seconde bride 25 lorsque le ou les tétons ont été amenés jusqu'au fond de leur rainure correspondante sur la tête de liaison 26.

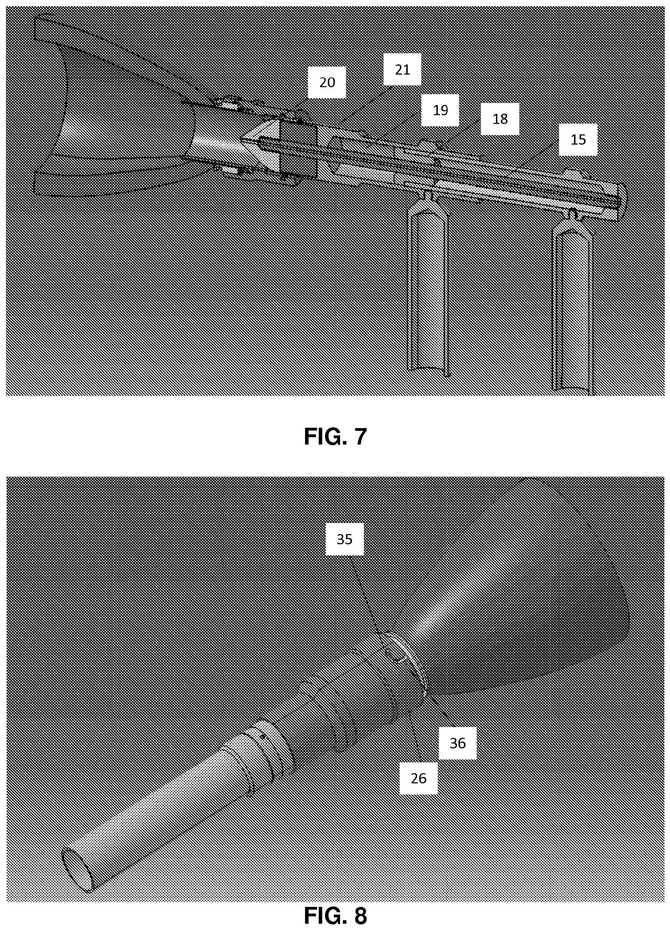

La Figure 7 est une vue en coupe du dispositif de carottage, la tête de carottage étant placée en retrait de l'ouverture réalisée dans la paroi ventriculaire, et de manière plus précise, étant placée dans le logement interne du corps du dispositif de carottage. Le fourreau 23 de l'accessoire d'assistance 22 a été introduit au travers de cette ouverture dans la chambre ventriculaire et la tête de liaison 26 est assemblée à la seconde bride 25 de la bague d'ancrage externe au cœur. Le praticien peut dès lors retirer le dispositif de carottage 10 sans risquer un déplacement de l'accessoire d'assistance 22 de l'ouverture réalisée dans la paroi ventriculaire.

Le fourreau 23 de cet accessoire d'assistance 22 permet de maintenir cette ouverture entièrement accessible pour assurer le libre passage de la partie de bague d'ancrage comprenant une portion tubulaire 27 et à l'extrémité distale de cette dernière, une membrane auto-expansible 24 dans sa première configuration.

Ce retrait du dispositif de carottage 10 entraîne la première perte d'étanchéité au niveau de la paroi ventriculaire de sorte que le praticien doit rapidement relier à cet accessoire creux, à un dispositif de poussée 28 portant cette partie de bague d'ancrage pour l'introduction de son extrémité dans la chambre ventriculaire 29.

Après avoir introduit la membrane auto-expansible 24 dans cette chambre ventriculaire, et cette dernière ayant adopté sa seconde configuration dans laquelle elle définit une bride, ou collerette, s'étendant radialement à partir de la portion tubulaire 27, le praticien recule le dispositif de poussée 28 pour venir plaquer cette bride contre la face intérieure de la paroi ventriculaire 29 du cœur battant.

Ainsi, les deux brides 24, 25 étant plaquées de part et d'autre contre cette paroi ventriculaire 29, le praticien n'a plus qu'à engager un élément d'étanchéité 30 tel qu'un joint d'étanchéité torique et un moyen de serrage 31 sur le dispositif de poussée 28 et faire coulisser ces derniers le long de la surface externe de ce dispositif de poussée 28 pour venir verrouiller en position la bague d'ancrage. Ce moyen de serrage 31 vient finaliser l'assemblage de la bague d'ancrage en bloquant tout déplacement des brides 24, 25 l'une par rapport à l'autre. On obtient ainsi une liaison mécanique résistante de la bague d'ancrage et de l'apex du cœur ainsi que de son étanchéité.

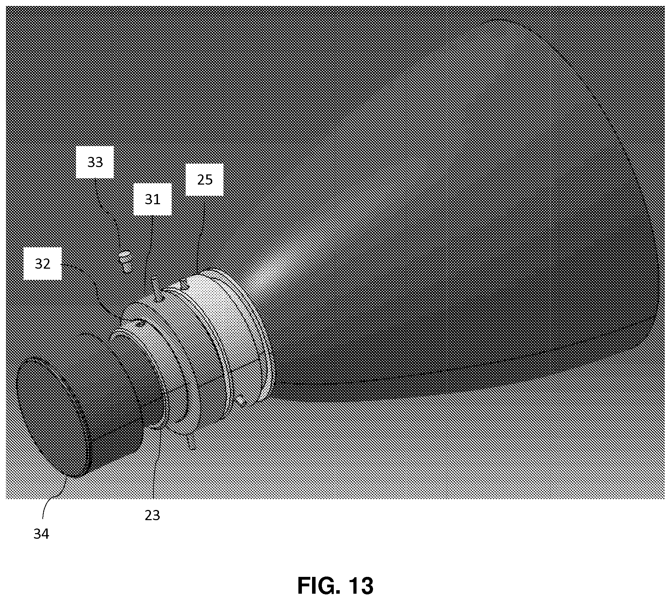

Ce moyen de serrage 31 peut être un écrou si la surface externe de la portion tubulaire 27, ou corps creux, comporte un filetage ou comme illustré à la Figure 13, une bague de serrage. Cette dernière vient presser l'élément d'étanchéité 30 contre la seconde bride 25 assemblée à la surface extérieure de la paroi ventriculaire 29.

Avantageusement, l'ensemble portion tubulaire 27/membrane auto-expansible 24 comprend un deuxième élément d'étanchéité comprenant une valve anti-reflux (non représentée) placée dans le canal interne délimité par cette portion tubulaire 27. A titre d'exemple, il s'agit d'une membrane auto réparatrice, dite encore membrane auto-seal, intégrée dans le canal interne délimité par la portion tubulaire 27, ou corps creux, de la bague d'ancrage. Cette valve anti-reflux empêche toute fuite de sang par ce canal interne. Elle permet ainsi, après retrait du dispositif de carottage, de rétablir l'étanchéité par la pose du dispositif d'ancrage sur le cœur.

A titre purement illustratif, cette valve anti-reflux est en silicone
Cette bague de serrage 31 comporte sur sa paroi latérale un orifice 32 recevant une fixation 33 pour verrouiller en position une pompe cardiaque 34 introduite dans le canal interne délimité par le corps creux de la bague d'ancrage, l'extrémité distale de cette pompe cardiaque 34 étant engagée dans chambre ventriculaire du cœur battant. Lors du retrait de la pompe cardiaque 34 du cœur, par exemple pour une manutention, reçue dans la portion tubulaire 27, ce second élément d'étanchéité se referme et redevient étanche.

## Revendications

1. Ensemble de pose d'un outil ou d'un accessoire chirurgical dans une paroi tissulaire comprenant:
- un dispositif de carottage pour réaliser une ouverture dans une paroi tissulaire, comprenant :
- un corps ayant un axe longitudinal, ledit corps ayant une extrémité proximale et une extrémité distale, ledit corps comportant un logement interne,
- une tête de carottage (13) comportant une lame de transsection (14), cette tête étant mobile entre une position de repos et une position actionnée, ladite tête de carottage ayant un axe de carottage,
- un mécanisme d'actionnement pour déplacer ladite tête entre lesdites positions, ledit mécanisme comportant une tige d'actionnement (15) à une extrémité de laquelle est placée ladite tête de carottage;
**caractérisé en ce que**
- l'extrémité distale dudit corps forme un support d'outil (21) chirurgical, ce support d'outil (21) est configuré pour
* d'une part, recevoir par emboîtement un outil ou accessoire chirurgical (22) creux et supporter cet outil ou accessoire, cet outil ou accessoire entourant l'extrémité distale dudit corps lorsqu'il est placé sur ledit support d'outil (21), en étant coaxial avec ledit axe de carottage, et
* d'autre part, permettre un guidage linéaire dudit outil ou accessoire chirurgical (22) lors de son déplacement depuis le dispositif de carottage vers l'ouverture réalisée dans la paroi tissulaire,
- ledit support d'outil (21) comporte une paroi périphérique externe qui est tubulaire et de diamètre égal ou sensiblement égal au diamètre de ladite lame de transsection (14), et
- ledit outil ou accessoire chirurgical (22) creux configuré pour être emboîté sur l'extrémité distale dudit dispositif de carottage tout en laissant ladite tête de carottage (13) dégagée, ledit outil ou accessoire chirurgical (22) comportant à son extrémité distale, une portion tubulaire ou sensiblement tubulaire configurée pour maintenir la forme et le diamètre de l'ouverture réalisée par carottage dans la paroi tissulaire, ladite portion tubulaire ou sensiblement tubulaire ayant une longueur au moins égale à l'épaisseur de la paroi tissulaire.

2. Ensemble de pose selon la revendication 1, **caractérisé en ce que** ledit dispositif comprend une lumière (20) s'étendant à travers au moins l'extrémité proximale dudit corps, ladite tige d'actionnement (15) et ladite tête de carottage (13) pour le passage d'un élément de guidage souple.

3. Ensemble de pose selon la revendication 1 ou 2, **caractérisé en ce que** ladite paroi tissulaire étant une paroi d'un cœur battant destiné à recevoir un dispositif d'ancrage d'une pompe cardiaque comprenant une bride, ou collerette, (25) destinée à être plaquée contre la surface extérieure de ladite paroi du cœur battant, la longueur dudit support d'outil (21) est au moins égale à la somme de l'épaisseur de ladite paroi tissulaire (29) et de l'épaisseur, ou dimension longitudinale, de ladite bride, ou collerette, (25) destinée à être placée contre la surface extérieure de la paroi dudit coeur; l'ensemble comportant ladite bride ou collerette (25).

4. Ensemble de pose selon l'une quelconque des revendications précédentes, **caractérisé en ce qu'**il comporte une partie d'un moyen détrompeur, ledit outil chirurgical (22) étant destiné à porter la partie complémentaire de ce moyen détrompeur, pour orienter ledit outil chirurgical (22) sur ledit support.

5. Ensemble de pose selon l'une quelconque des revendications précédentes, **caractérisé en ce qu'**il comporte une butée pour stopper le déplacement dudit outil chirurgical (22) le long dudit axe longitudinal et en direction de ladite extrémité proximale afin que l'outil chirurgical (22) reste positionné sur ledit support d'outil (21).

6. Ensemble de pose selon l'une quelconque des revendications précédentes, **caractérisé en ce que** ledit corps comporte deux parties de corps (11, 12) emboîtées l'une dans l'autre, une première partie de corps étant mobile en translation par rapport à l'autre partie de corps de sorte que son déplacement dans et hors de l'autre partie de corps provoque le déplacement en translation de ladite tête de carottage (13).

7. Ensemble de pose selon la revendication 6, **caractérisé en ce que** chaque partie de corps (11, 12) comporte une poignée de préhension (16, 17).

8. Ensemble de pose selon la revendication 6 ou 7, **caractérisé en ce que** ledit mécanisme d'actionnement comprend un chariot de guidage (18) supportant ladite tige d'actionnement (15) et reçu dans ledit logement interne (19), ce logement (19) étant configuré pour former un rail de guidage en translation dudit chariot de guidage (18), ledit chariot de guidage étant solidaire de ladite première partie de corps mobile en translation.

9. Ensemble de pose selon l'une quelconque des revendications précédentes, **caractérisé en ce que** le corps du dispositif de carottage comporte une partie d'un moyen détrompeur, ledit outil ou accessoire chirurgical (22) comportant une partie complémentaire de ce moyen détrompeur, pour orienter ledit outil ou accessoire chirurgical (22) sur ledit support d'outil (21).

10. Ensemble de pose selon l'une quelconque des revendications précédentes, **caractérisé en ce que** ledit outil ou accessoire chirurgical (22) est un outil d'assistance à la pose d'un dispositif d'ancrage d'une pompe cardiaque au travers d'une ouverture réalisée par carottage dans une paroi d'un cœur, ledit outil d'assistance creux délimitant un canal interne pour le passage dudit dispositif d'ancrage, dont le diamètre est sensiblement égal au diamètre de cette ouverture.

11. Ensemble de pose selon la revendication 10, comportant une bride, ou collerette, étant destinée à être placée contre la surface extérieure de la paroi dudit cœur, cette bride comprenant au moins une saillie, ou téton, placée sur sa face latérale, ledit outil d'assistance comporte pour chaque saillie, une rainure débouchant destinée à recevoir ladite saillie, ou téton, correspondante, ladite rainure étant configurée pour assurer par rotation dudit outil chirurgical (22) autour de l'axe de carottage, un verrouillage dudit outil chirurgical (22) sur ladite bride, ou collerette.

## Patentansprüche

1. Vorrichtung zum Anbringen eines chirurgischen Instruments oder Zubehörs in einer Gewebewand, umfassend:
- eine Coring-Vorrichtung zum Herstellen einer Öffnung in einer Gewebewand, umfassend:
- einen Körper mit einer Längsachse, wobei der Körper ein proximales Ende und ein distales Ende aufweist, wobei der Körper eine innere Aufnahme aufweist,
- einen Coring-Kopf (13), der eine Trennklinge (14) umfasst, wobei der Kopf zwischen einer Ruhestellung und einer Betätigungsstellung bewegbar ist, wobei der Coring-Kopf eine Coring-Achse aufweist,
- einen Betätigungsmechanismus zum Bewegen des Kopfes zwischen den Positionen, wobei der Mechanismus einen Betätigungsstab (15) aufweist, an dessen einem Ende der Coring-Kopf angeordnet ist; **dadurch gekennzeichnet, dass**
- das distale Ende des Körpers einen chirurgischen Werkzeughalter (21) bildet, wobei der Werkzeughalter (21) dazu ausgebildet ist,
* einerseits ein hohles chirurgisches Werkzeug oder Zubehör (22) durch Einstecken aufzunehmen und das Werkzeug oder Zubehör zu halten, wobei das Werkzeug oder Zubehör das distale Ende des Körpers umgibt, wenn es an der Werkzeughalterung (21) angeordnet ist, und koaxial zur Coring-Achse ist, und
* andererseits eine lineare Führung des chirurgischen Werkzeugs oder Zubehörs (22) bei seiner Verlagerung von der Coring-Vorrichtung zu der in der Gewebewand ausgebildeten Öffnung zu ermöglichen,
- wobei der Werkzeugträger (21) eine äußere Umfangswand aufweist, die rohrförmig ist und einen Durchmesser hat, der gleich oder im Wesentlichen gleich dem Durchmesser der Trennklinge (14) ist, und
- das hohle chirurgische Werkzeug oder Zubehör (22) derart ausgebildet ist, dass es auf das distale Ende der Coring-Vorrichtung aufgesteckt werden kann, wobei der Coring-Kopf (13) frei bleibt, wobei das chirurgische Werkzeug oder Zubehör (22) an seinem distalen Ende einen röhrenförmigen oder im Wesentlichen röhrenförmigen Abschnitt aufweist, der derart ausgebildet ist, dass er die Form und den Durchmesser der durch das Coring in der Gewebewand erzeugten Öffnung beibehält, wobei der röhrenförmige oder im Wesentlichen röhrenförmige Abschnitt eine Länge aufweist, die wenigstens der Dicke der Gewebewand entspricht.

2. Anbringvorrichtung nach Anspruch 1, **dadurch gekennzeichnet, dass** die Vorrichtung ein Lumen (20) umfasst, das sich durch wenigstens das proximale Ende des Körpers, den Betätigungsstab (15) und den Coring-Kopf (13) erstreckt, um ein flexibles Führungselement hindurchzuführen.

3. Anbringvorrichtung nach Anspruch 1 oder 2, **dadurch gekennzeichnet, dass** die Gewebewand eine Wand eines schlagenden Herzens ist, die dazu bestimmt ist, eine Verankerungsvorrichtung einer Herzpumpe aufzunehmen, die einen Flansch (25) umfasst, der dazu bestimmt ist, gegen die Außenfläche der Wand des schlagenden Herzens gedrückt zu werden, wobei die Länge des Werkzeugträgers (21) wenigstens gleich der Summe aus der Dicke der Gewebewand (29) und der Dicke oder Längsabmessung des Flansches oder Kragens (25) ist, der dazu bestimmt ist, an der Außenfläche der Wand des Herzens anzuliegen; wobei die Vorrichtung den Flansch oder Kragen (25) umfasst.

4. Anbringvorrichtung nach einem der vorhergehenden Ansprüche, **dadurch gekennzeichnet, dass** sie einen Teil einer Verwechslungssicherung umfasst, wobei das chirurgische Werkzeug (22) dazu bestimmt ist, den komplementären Teil dieser Verwechslungssicherung zu tragen, um das chirurgische Werkzeug (22) auf dem Träger auszurichten.

5. Anbringvorrichtung nach einem der vorhergehenden Ansprüche, **dadurch gekennzeichnet, dass** sie einen Anschlag aufweist, um die Verlagerung des chirurgischen Werkzeugs (22) entlang der Längsachse und in Richtung des proximalen Endes zu stoppen, damit das chirurgische Werkzeug (22) an dem Werkzeughalter (21) positioniert bleibt.

6. Anbringvorrichtung nach einem der vorhergehenden Ansprüche, **dadurch gekennzeichnet, dass** der Körper zwei ineinander gesteckte Körperteile (11, 12) umfasst, wobei ein erstes Körperteil gegenüber dem anderen Körperteil translatorisch beweglich ist, so dass seine Bewegung in das und aus dem anderen Körperteil eine Translationsbewegung des Coring-Kopfes (13) bewirkt.

7. Anbringvorrichtung nach Anspruch 6, **dadurch gekennzeichnet, dass** jedes Körperteil (11, 12) einen Griff (16, 17) aufweist.

8. Anbringvorrichtung nach Anspruch 6 oder 7, **dadurch gekennzeichnet, dass** der Betätigungsmechanismus einen Führungswagen (18) umfasst, der den Betätigungsstab (15) trägt und in dem inneren Gehäuse (19) aufgenommen ist, wobei das Gehäuse (19) derart ausgebildet ist, dass es eine Führungsschiene für die Translationsbewegung des Führungswagens (18) bildet, wobei der Führungswagen fest mit dem ersten translatorisch beweglichen Körperteil verbunden ist.

9. Anbringvorrichtung nach einem der vorhergehenden Ansprüche, **dadurch gekennzeichnet, dass** der Körper der Coring-Vorrichtung einen Teil einer Verwechslungssicherung umfasst, wobei das chirurgische Werkzeug oder Zubehör (22) einen komplementären Teil dieser Verwechslungssicherung umfasst, um das chirurgische Werkzeug oder Zubehör (22) auf dem Werkzeughalter (21) auszurichten.

10. Anbringvorrichtung nach einem der vorhergehenden Ansprüche, **dadurch gekennzeichnet, dass** das chirurgische Werkzeug oder Zubehör (22) ein Hilfswerkzeug zum Einbau einer Verankerungsvorrichtung einer Herzpumpe durch eine durch Coring hergestellte Öffnung in einer Herzwand ist, wobei das hohle Hilfswerkzeug einen Innenkanal für den Durchgang der Verankerungsvorrichtung begrenzt, dessen Durchmesser im Wesentlichen dem Durchmesser dieser Öffnung entspricht.

11. Anbringvorrichtung nach Anspruch 10, umfassend einen Flansch oder Kragen, der dazu bestimmt ist, an der Außenfläche der Wand des Herzens platziert zu werden, wobei dieser Flansch wenigstens einen Vorsprung oder Zapfen aufweist, der an seiner Seitenfläche angeordnet ist, wobei das Hilfswerkzeug für jeden Vorsprung eine durchgehende Nut aufweist, die dazu bestimmt ist, den entsprechenden Vorsprung oder Zapfen aufzunehmen, wobei die Nut derart ausgebildet ist, dass sie durch Drehen des chirurgischen Werkzeugs (22) um die Coring-Achse eine Verriegelung des chirurgischen Werkzeugs (22) an dem Flansch oder Kragen gewährleistet.

## Claims

1. Assembly for fitting a surgical tool or accessory in a tissue wall comprising:
- a coring device for forming an opening in a tissue wall, comprising:
- a body having a longitudinal axis, said body having a proximal end and a distal end, said body comprising an inner housing,
- a coring head (13) comprising a transection blade (14), this head being movable between a rest position and an actuated position, said coring head having a coring axis,
- an actuation mechanism for moving said head between said positions, said mechanism including an actuation rod (15) at one end of which said coring head is placed;
**characterised in that**
- the distal end of said body forms a surgical tool support (21), this tool support (21) is configured
* on the one hand, to receive by engagement a hollow surgical tool or accessory (22) and to support this tool or accessory, this tool or accessory surrounding the distal end of said body when it is placed on said tool support (21), by being coaxial with said coring axis, and,
* on the other hand, to enable a linear guidance of said surgical tool or accessory (22) during the movement thereof from the coring device towards the opening formed in the tissue wall,
- said tool support (21) comprises an outer peripheral wall which is tubular and has a diameter equal to or substantially equal to the diameter of said transection blade (14), and
- said hollow surgical tool or accessory (22) configured to be engaged on the distal end of said coring device while leaving said coring head (13) exposed, said surgical tool or accessory (22) including at the distal end thereof, a tubular or substantially tubular portion configured to maintain the shape and the diameter of the opening formed by coring in the tissue wall, said tubular or substantially tubular portion having a length at least equal to the thickness of the tissue wall.

2. Fitting assembly according to claim 1, **characterised in that** said device comprises a lumen (20) extending through at least the proximal end of said body, said actuation rod (15) and said coring head (13) for the passage of a flexible guide element.

3. Fitting assembly according to claim 1 or 2, **characterised in that** said tissue wall being a wall of a beating heart intended to receive a device for anchoring a cardiac pump comprising a flange, or collar, (25) intended to be pressed against the outer surface of said wall of the beating heart, the length of said tool support (21) is at least equal to the sum of the thickness of said tissue wall (29) and the thickness, or longitudinal dimension, of said flange, or collar, (25) intended to be placed against the outer surface of the wall of said heart; the assembly including said flange or collar (25).

4. Fitting assembly according to any one of the preceding claims, **characterised in that** it includes a part of a foolproof means, said surgical tool (22) being intended to carry the complementary part of this foolproof means, to orient said surgical tool (22) on said support.

5. Fitting assembly according to any one of the preceding claims, **characterised in that** it includes a stop to stop the movement of said surgical tool (22) along said longitudinal axis and in the direction of said proximal end so that the surgical tool (22) remains positioned on said tool support (21).

6. Fitting assembly according to any one of the preceding claims, **characterised in that** said body includes two body parts (11, 12) engaged with one another, a first body part being movable in translation in relation to the other body part so that the movement thereof in and out of the other body part causes said coring head (13) to move in translation.

7. Fitting assembly according to claim 6, **characterised in that** each body part (11, 12) comprises a gripping handle (16, 17).

8. Fitting assembly according to claim 6 or 7, **characterised in that** said actuation mechanism comprises a guide carriage (18) supporting said actuation rod (15) and received in said inner housing (19), this housing (19) being configured to form a translational guide rail of said guide carriage (18), said guide carriage being integral with said first body part movable in translation.

9. Fitting assembly according to any one of the preceding claims, **characterised in that** the body of the coring device includes a part of a foolproof means, said surgical tool or accessory (22) including a complementary part of this foolproof means, for orienting said surgical tool or accessory (22) on said tool support (21).

10. Fitting assembly according to any one of the preceding claims, **characterised in that** said surgical tool or accessory (22) is a tool for assisting the fitting of a device for anchoring a cardiac pump through an opening formed by coring in a wall of a heart, said hollow assistance tool delimiting an internal channel for the passage of said anchoring device, the diameter of which is substantially equal to the diameter of this opening.

11. Fitting assembly according to claim 10, including a flange, or collar, being intended to be placed against the outer surface of the wall of said heart, this flange comprising at least one protrusion, or stud, placed on the lateral face thereof, said assistance tool includes for each protrusion, an opening groove intended to receive said corresponding protrusion, or stud, said groove being configured to ensure by rotating said surgical tool (22) about the coring axis, locking said surgical tool (22) on said flange, or collar.
